# EUROPÄISCHE PATENTANMELDUNG

(11) **EP 0 792 864 A1**
(43) Veröffentlichungstag der Anmeldung: **03.09.1997**
(21) Anmeldenummer: 97102842.8
(22) Anmeldetag: 21.02.1997
(51) Int. Cl.: C07C 49/523, C07C 45/49

(54) **4-Formyl-3,3,5-trimethyl-cyclohexanon und Verfahren zu seiner Herstellung**

(30) Priorität: 01.03.1996 DE 19607957
(71) Anmelder: BASF AKTIENGESELLSCHAFT, 67056 Ludwigshafen (DE)
(72) Erfinder: Langguth, Ernst, Dr., 67281 Kirchheim (DE); Röper, Michael, Prof,-Dr., 67157 Wachenheim (DE); Zeller, Edgar, Dr., 68163 Mannheim (DE)

(57) **Zusammenfassung**

4-Formyl-3,3,5-trimethyl-cyclohexanon und dessen Herstellung durch Hydroformylierung von β-Isophoron.

## Beschreibung

Die Erfindung betrifft 4-Formyl-3,3,5-trimethyl-cyclohexanon der Formel I 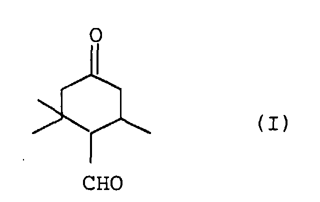 sowie ein Verfahren zu seiner Herstellung.

Das 4-Formyl-3,3,5-trimethyl-cyclohexanon ist als potentielles Zwischenprodukt für Carotinoid- oder Riechstoffsynthesen von Interesse.

Beispielsweise könnte das aus der Formylverbindung I herstellbare 4-Ethoxycarbonyl-3,3,5-trimethyl-cyclohexanon zur Herstellung der begehrten Riechstoffe δ- und ε-Damascon in dem Verfahren gemäß DE 32 33 175 verwendet werden.

Es wurde überraschenderweise gefunden, daß sich β-Isophoron in Gegenwart von Rhodiumkatalysatoren bei erhöhtem Druck und erhöhten Temperaturen hydroformylieren läßt, während bei α-Isophoron unter den Reaktionsbedingungen einer Hydroformylierung nahezu ausschließlich eine Hydrierung der C-C-Doppelbindung eintritt. Die bei der Hydroformylierung von β-Isophoron als Hauptprodukt anfallende Verbindung konnte durch Destillation gereinigt werden. Das gereinigte Produkt erwies sich durch GC/MS- und NMR-Analyse als 4-Formyl-3,3,5-trimethyl-cyclohexanon der Formel I. Es weist einen Siedepunkt von 84°C/1 mbar auf.

Gegenstand der Erfindung ist daher neben 4-Formyl-3,3,5-trimethyl-cyclohexanon der Formel I ein Verfahren zu seiner Herstellung, das dadurch gekennzeichnet ist, daß man β-Isophoron der Formel II 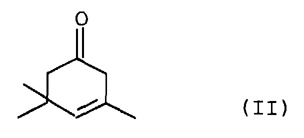 in Gegenwart von Rhodium-Hydroformylierungskatalysatoren bei erhöhtem Druck und erhöhten Temperaturen mit einem Gemisch aus Kohlenmonoxid und Wasserstoff hydroformyliert.

Die Hydroformylierung des β-Isophorons kann durch Umsetzen mit einem Gemisch aus CO und H₂ unter erhöhtem Druck in Gegenwart eines Hydroformylierungskatalysators und ggf. in Gegenwart eines inerten Lösungsmittels bei erhöhter Temperatur und längeren Reaktionszeiten erfolgen.

CO und H₂ verwendet man hierbei im allgemeinen in einem Partialdruckverhältnis, und damit in einem Molverhältnis von 0,1 zu 1 bis 10 zu 1, vorzugsweise etwa 0,8:1 bis 1,2:1, insbesondere 1:1.

Der Druck im geschlossenen Reaktionsgefäß liegt im allgemeinen bei 50 bis 1000 bar, vorzugsweise 280 bis 600 bar.

Als Hydroformylierungskatalysatoren kommen homogen im Hydroformylierungsmedium gelöste Rhodiumverbindungen in Betracht, die vorzugsweise nicht mit phosphorhaltigen Liganden wie Phosphinen oder Phosphiten modifiziert sind. Die wirksamen Rhodium-Hydroformylierungskatalysatoren bilden sich unter der Bedingungen der Hydroformylierungsreaktion im Reaktionsgemisch aus Rhodium-Verbindungen z.B. Rhodiumsalzen, wie Rhodium(III)chlorid, Rhodium(III)sulfat, Rhodium(III)nitrat, Rhodium(III)acetat, Rhodium(III)acetylacetonat, Rhodium(III)sulfat, Rhodium(III)ammoniumchlorid, Rhodiumchalkogeniden, wie Rhodium(III)oxid oder Rhodium(III)sulfid, Salzen aus Rhodiumsauerstoffsäuren, wie den Rhodaten oder aus Rhodiumcarbonylverbindungen, wie Rhodiumdicarbonyl-acetylacetonat, Cyclooctadien-Rhodiumacetat oder Cyclooctadien-Rhodiumchlorid.

Den Rhodium-Hydroformylierungskatalysator verwendet man im allgemeinen in Mengen von 200 bis 2000, vorzugsweise 400 bis 1000 mg/kg des β-Isophorons (Rhodium berechnet als Metall).

Als inerte Lösungsmittel kommen solche in Betracht, die unter den Reaktionsbedingungen inert sind und sowohl den Hydroformylierungskatalysator als auch das β-Isophoron möglichst gut lösen. Genannt seien beispielsweise gesättigte Kohlenwasserstoffe, wie Hexan, Octan, Dodecan, Decalin, Tetrahydronaphthalin, Cyclohexan, Cycloheptan oder Alkylcycloalkane; aromatische Kohlenwasserstoffe wie Benzol, Toluol oder Xylol; Ether, wie Tetrahydrofuran, Tetrahydropyran, Diethylether, 1,2-Dimethyoxy-benzol, 1,2-Diethoxybenzol sowie Mono- oder Diether des Ethylenglykols, Propylenglykols, Butylenglykols, Diethylenglykols oder Dipropylenglykols; inerte fluorierte Kohlenwasserstoffe, wie Perfluorethan oder Nonofluorbenzol; oder aber geeignete Mischungen aus den genannten Lösungsmitteln.

Mit besonderem Vorteil verwendet man als Lösungsmittel Toluol.

Das Lösungsmittel verwendet man im allgemeinen in Mengen von etwa 0,5 bis 2, vorzugsweise 0,8 bis 1,2 kg pro kg β-Isophoron.

Die Hydroformylierung wird im allgemeinen bei Temperaturen von etwa 50 bis 150°C, vorzugsweise 80 bis 120°C durchgeführt.

Je nach der Reaktionstemperatur müssen Reaktionszeiten von 5 bis. 100 Stunden, vorzugsweise 15 bis 75 Stunden angewendet werden.

Um möglichst gute Ausbeuten an dem Hydroformylierungsprodukt I zu erzielen, muß man unter Bedingungen arbeiten, unter denen die Hydrierung und die Isomerisierung möglichst gering sind.

Der häufig bei Hydroformylierungen übliche Zusatz von nennenswerten Mengen an Phosphiten, wie P(C₆H₅O)₃, bringt bei dieser Umsetzung keinen Vorteil sondern eher Nachteile, da hierdurch - wider Erwarten - die Isomerisierung von β-Isophoron zu dem nicht hydroformylierbaren α-Isophoron beschleunigt wird.

Das aus dem technisch leicht verfügbaren β-Isophoron durch Hydroformylierung erhältliche 4-Formyl-3,3,5-trimethyl-cyclohexanon kann als Zwischenprodukt für Carotinoid- oder Riechstoffsynthesen Bedeutung gewinnen. Außerdem kann man aus ihm durch einfache Folgereaktionen interessante Alkohole, Säuren und Amine erhalten.

### Beispiele 1 bis 8 (Beispiele 1 und 8 sind Vergleichsbeispiele)

In einem 0,3 Liter Autoklaven wurden jeweils 75 g β-Isophoron in 75 g Toluol in Gegenwart der aus der folgenden Tabelle ersichtlichen Mengen an dem Hydroformylierungskatalysator in Form von Rh(CO)₂ acetylacetonat (Rh) und der aus der Tabelle ersichtlichen Menge an P(C₆H₅O)₃ mit einem CO/H₂-Gemisch im Verhältnis 1:1 unter dem aus der Tabelle ersichtlichen Druck für die aus der Tabelle ersichtliche Reaktionszeit (RkZ) in Beispiel 1 auf 150°C und bei den übrigen Beispielen auf 100°C erhitzt.

In der Tabelle sind die jeweils erzielten Umsätze, die Selektivitäten für die Hydrierung (Hydrier.) zu 3,3,5-Trimethyl-cyclohexanon und die Isomerisierung (Isomer.) zu α-Isophoron sowie die Selektivitäten und Ausbeuten für die Hydroformylierung zu 4-Formyl-3,3,5-trimethyl-cyclohexanon der Formel I angegeben. Mit Hilfe von Spektrensimulationen sowie ein- und zweidimensionalen NMR-Methoden konnte die Struktur des Hydroformylierungsproduktes I aufgeklärt werden. Es handelt sich um trans-3,3-Dimethyl-4-formyl-5-methyl-cyclohexanon. Die destillativ gereinigte Verbindung weist einen Siedepunkt von 84°C bei 1 mbar Druck auf. Die Umsätze wurden durch Gaschromatographie (Gc) bestimmt.

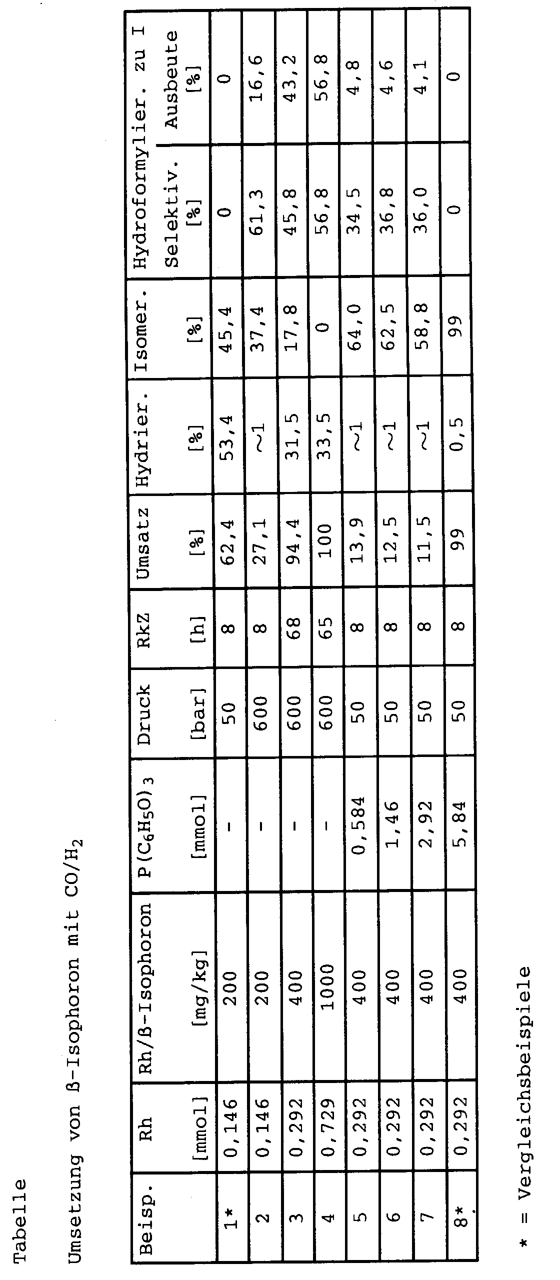

## Patentansprüche

1. 4-Formyl-3,3,5-trimethyl-cyclohexanon der Formel I

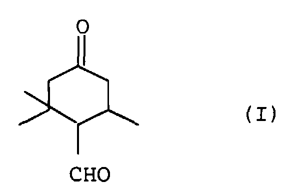

2. Verfahren zur Herstellung von 4-Formyl-3,3,5-trimethyl-cyclohexanon der Formel I, dadurch gekennzeichnet, daß man β-Isophoron der Formel II 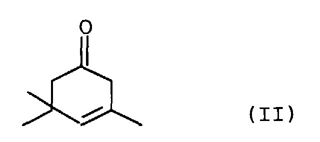 in Gegenwart von Rhodium-Hydroformylierungskatalysatoren bei erhöhtem Druck und erhöhten Temperaturen mit einem Gemisch aus Kohlenmonoxid und Wasserstoff hydroformyliert.
